# EUROPEAN PATENT SPECIFICATION

(11) **EP 3 888 538 B1**
(45) Date of publication and mention of the grant of the patent: **05.06.2024**
(21) Application number: 20167546.9
(22) Date of filing: 01.04.2020
(51) Int. Cl.: A61B 5/021, A61B 5/024, A61B 5/00

(54) **SMART COVER FOR SENSING A SIGNAL AND METHOD TO OPERATE THE SMART COVER**
INTELLIGENTE ABDECKUNG ZUM ERFASSEN EINES SIGNALS UND VERFAHREN ZUM BETREIBEN DER INTELLIGENTEN ABDECKUNG
COUVERCLE INTELLIGENT POUR LA DÉTECTION D'UN SIGNAL ET PROCÉDÉ DE FONCTIONNEMENT DU COUVERCLE INTELLIGENT

(43) Date of publication of application: 06.10.2021
(73) Proprietor: Benecke-Kaliko AG, 30419 Hannover (DE)
(72) Inventor: Damodharan, Suchitra, 30419 Hannover (DE)
(74) Representative: Continental Corporation

(56) References cited:
- US-A1- 2007 156 031
- US-A1- 2014 361 871
- US-A1- 2016 200 220
- US-A1- 2016 278 709
- US-A1- 2019 381 860
- US-B1- 10 299 520

## Description

The present invention relates to a smart cover for sensing a signal and to a method to operate the smart cover. The invention is described with respect to an automobile having a seat and a steering device but may be advantageously used with any other chair or with gym equipment having a seat and a handle or in hospitals & clinics.

Some vehicles have one or more seats, a steering device and at least one safety system which aims to increase driver and passenger safety. Some of these safety systems should function better if they could use data referring to the driver or passenger (occupant).

US 10,299,520 B 1 discloses a fabric-based item, which may adapat to and adjust the biometric state of an individual that is wearing or touching the fabric-based item. The fabric-based item may include one or more sensors that gather biometric information about the individual and one or more environmental control devices that adjust or maintain the environment around the individual based on the biometric information.

US 2019/0381860 A1 describes methods and apparatus relating to optimization of vehicular systems for occupant presence and condition. A logic circuitry is present to transmit a request to the vehicle to cause an adjustment to one or more function of the vehicle based at least in part on stored information.

In US 2014/0361871 A1 a device and method for continuous biometric recognition based on electrocardiographic signals ist described.

US 2016/0200220 A1 describes a seat, which comprises a cover member having a contact surface for a seated occupant, a a capacitive coupling sensor disposed opposite to the contact surface and configured to detect through the cover member, when the seated occupant is seated, a body potential of the seated occupant, and to output a signal correspondingto the body potential. A portion of the seat facing the sensor comprises a dielectric configured to increase a dielectrict constant of the portion.

US 2016/0278709 A1 relates to a piezoresistive textile sensor for detecting the heart and respiratory rate.

It is an objective of the present invention to provide more safety to the occupant of a vehicle.

The object is achieved by a smart cover according to claim 1 and by a method to operate the smart cover (second aspect).

The smart cover according to the first aspect is arranged for sensing a signal. The smart cover comprises a preferably conducting sheet of fabric or leather arranged to cover a seat, a handle and/or a steering device particularly of a vehicle, and a sensor matrix printed on the sheet and comprising several sensor elements. Each of the sensor elements is arranged for sensing or detecting an electrical signal sent out by a body of a human occupant, an outline of the body, and a body dimension.

The method according to the second aspect is suitable to operate a smart cover. The method comprises the following steps:
S2 sensing or detecting by sensor elements (4) one or more of an electrical signal sent out by a body of a human occupant, an outline of the body, and a body dimension, wherein the sensor elements are arranged in a sensor matrix of the smart cover, which sensor matrix is printed on a preferably conductive sheet of fabric or leather of the smart cover, wherein the sheet is arranged to cover a seat or a steering device of a vehicle, particularly to detect the presence of the occupant and/or to provide occupant presence information, further comprising one or both of the following steps
S3 estimating the physique of the occupant based on the signals received by the sensor elements, and providing respective anthropometric data, and
S5 increasing the sensitivity of at least one of the sensor elements according to data of step S3, and
S6' obtaining the physiologic parameter of the occupant, namely ECG, GSR or EEG, based on the signals received by the sensor elements, using the at least one sensor element of step S5.

Health Monitoring of driver or occupants in vehicle or automotive environment has been the trend for a long time. The usual ways to get access to the health of the occupants is to one, connect and synchronize activity trackers; two, connect ambulatory health monitoring systems, and three, use of bio vital acquiring sensors within the vehicle.

Although the best and easiest way to access the health is the third option, the sensors used for this application need to be placed on the location of body that is the target location of sensor. For e.g. blood pulse volume/rate can be acquired from finger tips, ear lobes or centre in the back of torso. Hence there is a need to identify and recognize the target locations on which the sensor can read the required information.

The invention permits to detect the physique, a seating position of the occupant and/or continuous monitoring of one of the occupant's physiologic parameters, such as heart activity. Thereby, a safety system can adapt to the detected physique of the occupant. There is no need for the occupant to key in data prior to driving or riding the vehicle. In case of an undesirable physiologic parameter a safety system or comfort system could adapt based on the physiologic parameter, at least a warning could be given to the occupant.

The invention helps to ensure that the safety systems depended on occupant or driver health work effectively for every seating position, contour and kind.

The body dimension can relate to one or more of armpit, crotch, shoulders, neck waist, chest, hips.

Within the concept of the present invention, physiologic parameters are particularly the heart's beats per minute, heart rate variability, blood pressure, blood oxygen saturation, breathing cycles per minute, the tidal volume per breathing cycle and characteristic values in an ECG.

Preferred embodiments given below may be combined advantageously, unless stated otherwise.

The sensor matrix of an embodiment is made with capacitive sensor elements. Each of the capacitive sensor elements can comprise two electrodes which are isolated from each other and which are printed on the sheet. The occupant can serve to increase the capacitance C of the capacitive sensor element including the two isolated electrodes. The printed electrodes can have a rounded or essentially rectangular shape or the shape of a cross. The two printed electrodes belonging to the same capacitive sensor element need not be immediately adjacent to each other. One of the capacitive sensor elements can form temporarily, when the electronic control unit described below relates a first and a second of the printed electrodes temporarily.

In case the sensor matrix is of the capacitive technology, the EEG and ECG measurements are feasible with increased resolution of these capacitive sensors. A derived combination of sensors in the matrix according to the anthropometric data/information leads to accurate EEG and ECG information. With processing these signals can provide hear rate, heart rate variability and insights on cardiac abnormalities. Additionally, for PPG (blood volume/rate), blood glucose etc. the sensing technology specific to these bio vitals are aligned, rotated or moved and activated with respect to the detected contour. The EEG signals are processed and analysed along with ECG signal characteristics to estimate the mental health like stress, distraction, drowsiness etc. and emotional health like mood, frustration, road rash etc.

The sensor matrix can be of any technology that can individually sense the body touch / contact to establish the outline contour with local extreme points (minimum and maximum vertical and horizontal coordinates). Optical, capacitive and force sensitive resistors are possible.

The sensing technology used for sensor matrix can/not be utilized for sensing the physiological signals. In case of capacitive sensors, additionally ECG, GSR and EEG signals can be acquired. In case of optical and piezo sensors, PPG signals can be acquired.

The sensor matrix is arranged to detect the occupant's physique, based on the signals received by the sensor elements, and is arranged to provide an anthropometric data based on the signals received by the sensor elements, particularly to a safety system of a vehicle.

In an embodiment, a first group of sensor elements is positioned and can be activated to detect electric heart signals of the occupant. The smart cover can indicate to a safety system whether the occupant is overloaded or stressed or tired.

The sensor matrix can be of any technology that can individually sense the body touch / contact to establish the outline contour with local extreme points (minimum and maximum vertical and horizontal coordinates). Optical, capacitive and force sensitive resistors are possible.

The sensing technology used for sensor matrix can/not be utilized for sensing the physiological signals. In case of capacitive, additionally ECG, GSR and EEG signals can be acquired. In case of optical and piezo, PPG signals can be acquired.

The sheet of another embodiment is arranged to cover a handle or steering device. Some of the sensor elements are positioned and arranged to detect or provide a dual electrode ECG from the occupant's fingers placed on the handle or steering device. Signals from the fingers of the right & left hand placed on the handle or steering device can be used advantageously for the dual electrode ECG.

The sheet of a further embodiment is arranged to cover the seat particularly of a vehicle or gym equipment. Some of the sensor elements can be positioned and arranged to detect or provide a 12 electrode ECG after/by estimating the location of exact electrode positions from the electrically generated profile or anthropometric data.

According to the invention, the smart cover has an electronic control unit arranged to process signals from the sensor elements into anthropometric data and a physiologic parameter of the occupant. The control unit can be arranged to provide the anthropometric data and the physiologic parameter to an adaptive safety system, a predictive safety system and/or to a comfort system particularly of a vehicle. The control unit can have an independent power supply unit providing power also to the sensor matrix. The control unit is arranged to process the signals from the sensor elements into one of evaluated physiologic parameters ECG, GSR and EEG. The control unit may employ an array for storing signals of several sensor elements at different points in time. The smart cover's electronic control unit can be arranged for wireless communication with a superordinate control unit e.g. of a vehicle.

A preferred vehicle has a smart cover as explained above and a safety system, preferably an adaptive passive safety system. The smart cover can be connected with the safety system to provide it with anthropometric data and a physiologic parameter of the occupant. The safety system can be arranged to operate based on the anthropometric data and the physiologic parameter. The vehicle can have a comfort system and/or a predictive safety system, each arranged to operate based on the anthropometric data and/or the physiologic parameter. The smart cover can have the control unit which begins to operate once the vehicle's ignition is switched on. The vehicle can be an automobile, lorry, locomotive, bicycle, motorcycle, ship or airplane. The smart cover can be part of a seat cover.

The smart cover can be used with gym equipment having a seat and/or a handle bar. The smart cover can cover the handle bar such that the hands of a user can touch sensor elements of the sensor matrix. The electronic control unit of the smart cover can be supplied with power by the gym equipment. The electronic control unit of the smart cover can be connected with the gym equipment such that a load on the user can be varied depending on a physiologic parameter of the user, particularly to avoid an overload. The electronic control unit can be connected with the gym equipment such that a member of the gym equipment can be positioned depending on the anthropometric data. This can also serve to avoid an accident.

The smart cover can be part of a chair at home or in an office or part of an arm chair. The smart cover can be used in hospitals & clinics. When the smart cover is part of a bed or pillow sheet or forms a bed or pillow sheet, a patient or an elderly person can be monitored. A physiologic parameter of the patient/person can be derived from the signals from the capacitive sensor elements. Movements of the patient/person can be detected based on changes of the signals from the capacitive sensor elements. If a physiologic parameter develops undesirably, then a countermeasure can be initiated or at least a warning can be given.

The system can facilitate a subject and his/her position independent physiology, health and state monitoring of the occupant in an automotive environment. The system facilitates use of sensors interconnected as a matrix embedded or printed as electronics on seat cover and steering wheel cover. As an occupant is seated, his contour outline is sensed using the sensor matrix and related anthropometric information is gained. For ECG and EEG, the same sensors/sensing technology is applicable, hence gets activated on the location estimated from the contour in the sensor matrix. The sensors can be also exclusive, independent of sensor matrix (for e.g. glucose, blood pulse etc.) and are additionally activated based on the contour detected.

An embodiment of the method comprises the step of activating/switching on the smart cover (S 1), particularly when switching on an ignition device of a vehicle or when activating a piece of gym equipment. This can help to save energy and/or to relieve the occupant/user from keying in data.

Another embodiment of the method includes activating or adjusting an adaptive passive safety system of the vehicle based on the anthropometric data of step S3 (S4). This can help to increase occupant safety.

An embodiment of the method involves activating a predictive safety system and/or a comfort system based on the physiologic parameter of step S6 or step S6' (S7).

A further embodiment includes a step of
S8 monitoring changes over time of at least one physiologic parameter of the occupant.

To this end, two subsequent signals of the same sensor element can be compared. It may be useful to compare a signal of one of the sensor elements at a first point in time with the signal of the same sensor element at a second point in time, wherein Δt ≥ N minutes and N is a natural number or positive integer greater than 0. The control unit of the smart cover may employ an array for storing signals of several sensor elements at different points in time. When comparing signals of the same sensor at different points in time, the control unit may access the array.

Another embodiment includes the step of
S9 monitoring changes over time of the electrically generated profile or anthropometric data of the occupant.

To this end, two subsequent signals of the same sensor element can be compared. It may be useful to compare a signal of one of the sensor elements at a first point in time with the signal of the same sensor element at a second point in time, wherein Δt ≥ N minutes and N is a natural number or positive integer greater than 0. A changing electrically generated profile or "changing anthropometric data" may indicate that the posture of the occupant changes indicating discomfort, pain or fatigue.

One of the smart covers explained above can advantageously be operated as follows:
S11 switching one or more sensors of the sensor matrix individually, preferably adapting one or more switched sensors,
S12 acquiring signals from the sensor matrix, preferably converting the acquired signals into digital and/or filtering the acquired signals (processed signals),
S13 communicating or transferring acquired or processed signals to an electronic control unit,
S14 determining minimum and maximum extreme points from the transfer to data by the control unit,
S15 identifying a contour of a body engaging with the sensor matrix,
S16 estimating anthropometric data referring to the body,
S17 estimating a sensing area for physiological signals referring to the body,
S18 deciding or selecting sensors to be activated and preparing instruction for configuring these sensors based on the estimated sensing area,
S19 communicating or transferring the instructions prepared during step S18 to a physiology sensor unit,
S20 activating sensors of the sensor matrix with the instruction of steps S18, S19,
S21 acquiring signals by the activated sensors,
S22 verifying the accuracy of the acquired physiology signals and verifying signal characteristics,
S23 returning the verified physiology signals to the control unit.

Further details and advantages are apparent to the skilled person from the following exemplary embodiment.

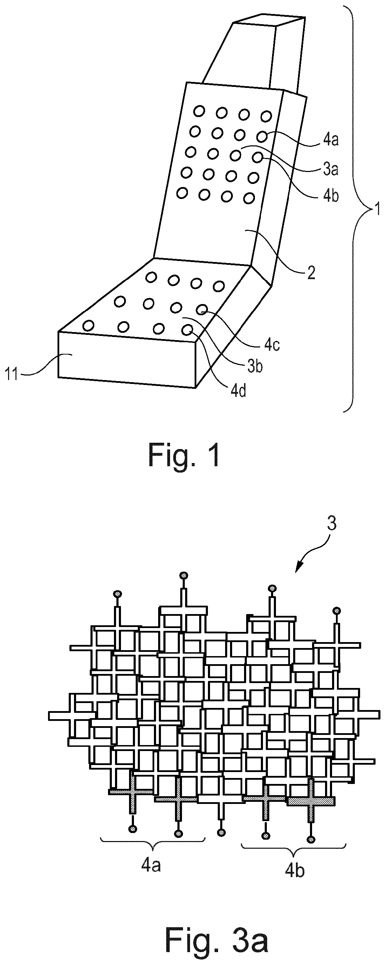

Figure 1 schematically shows a smart cover 1 which covers an independent car seat 11. The smart cover has a fabric sheet 2 on which a sensor matrix 3a, 3b is printed. The smart cover's sheet can be sewn to other cuttings to be a part of the same seat cover. The sensor matrix has several capacitive sensor elements 4a to 4d. The smart cover can have electronic control unit receiving and processing the sensor signals into anthropometric data and/or a physiologic parameter relating to the occupant. The electronic control unit of the smart cover can provide the anthropometric data and/or the physiologic parameter to a safety system of the independent car. Further, the electronic control unit can begin to operate when the ignition of the car is switched on.

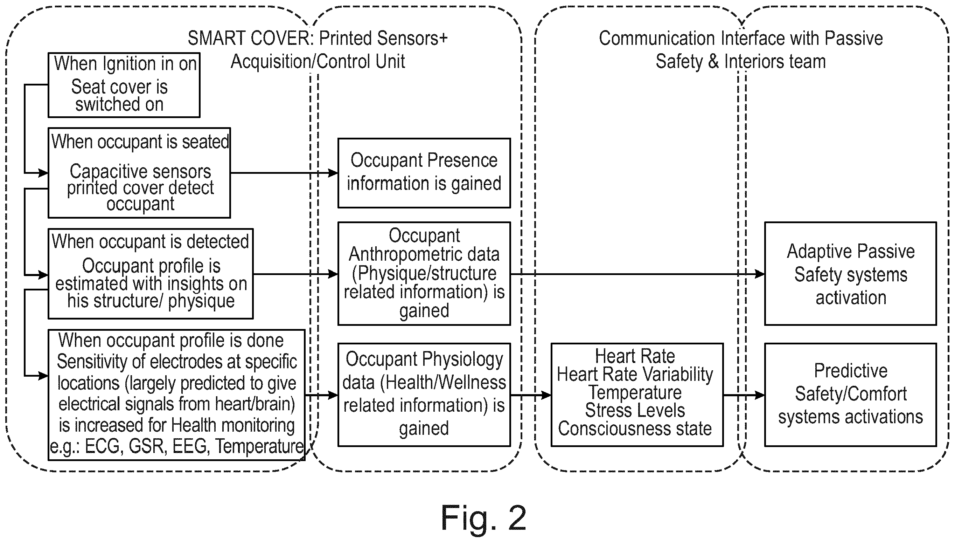

Figure 2 shows how an exemplary smart cover can be operated.

The smart cover can be switched on when switching on an ignition device of the vehicle having the smart cover (S 1). Step S2 is performed including sensing or detecting an electrical signal sent out by a body of a human occupant by sensor elements arranged in a sensor matrix of the smart cover, which sensor matrix is printed on a preferably conductive sheet of fabric or leather of the smart cover, wherein the sheet is arranged to cover a seat or a steering device of a vehicle. Thereby, the occupant is detected and occupant presence information 23 can be provided.

Afterwards, one or both of the following steps are executed:
S3 estimating the physique of the occupant based on the signals received by the sensor elements, and providing respective anthropometric data 21, and/or
S6 obtaining a physiologic parameter 22 of the occupant, such as ECG, GSR, EEG (evaluated physiologic parameter) or temperature 24, based on the signals received by the sensor elements.

The smart cover can provide a safety system of a vehicle with anthropometric data 21 (obtained by S3) an/or a physiologic parameter (obtained by S6) referring to the occupant. An adaptive passive safety system of a vehicle can be activated or adjusted based on the anthropometric data obtained by step S3 (S4).

The smart cover can be operated such that the sensitivity of at least one of the sensor elements is increased (S5). During step S5, a first group of sensor elements can be activated to detect electric heart signals of the occupant. Using the at least one sensor element of step S5, the physiologic parameter of the occupant, such as ECG, GSR, EEG or temperature, can be obtained based on the signals received by the sensor elements (S6').

A predictive safety system and/or a comfort system can be activated (S7) based on the physiologic parameter of step S6 or step S6'.

As shown by Figure 3a, several capacitive sensor elements 4a, 4b of a sensor matrix 3 are shown schematically. Each of the capacitive sensor elements 4a, 4b includes two electrodes (cross shaped, marked black) which are electrically isolated from each other. Each of the electrodes may have an individual electrical contact for connecting with an electronic control unit of the smart cover. Further capacitive sensor elements of the sensor matrix and the sheet, on which the sensor matrix is printed, are not shown. Two of the electrodes may form a capacitive sensor element, temporarily.

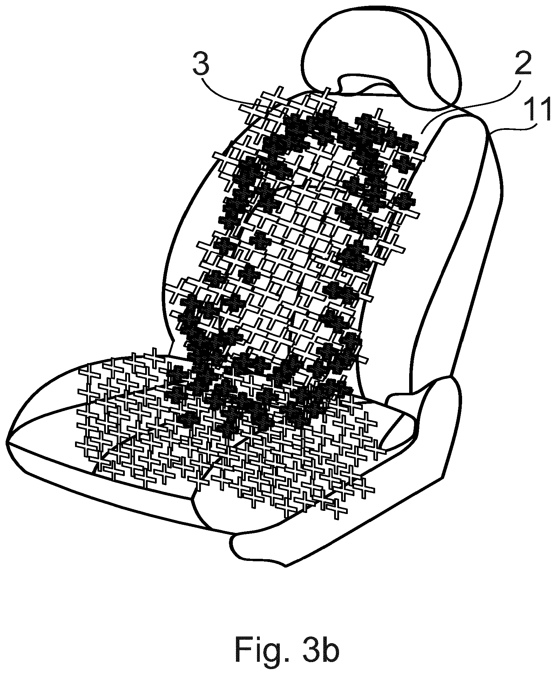

Figure 3b schematically shows a car seat 11 having the exemplary smart cover 1. The smart cover comprises the sensor matrix as is shown in Figure 3a. The sensors are each shown in a cross shape but they can have any other shape. The smart cover is part of the car seat as its sheet 2 is sewn to other cuttings to form the seat cover. Some of the capacitive sensor elements are shown in red/dark while others are shown in a lighter colour. Signals of the capacitive sensor elements shown in red/dark can be read and interpreted to provide anthropometric data, occupant presence information and the position of the occupant on the seat.

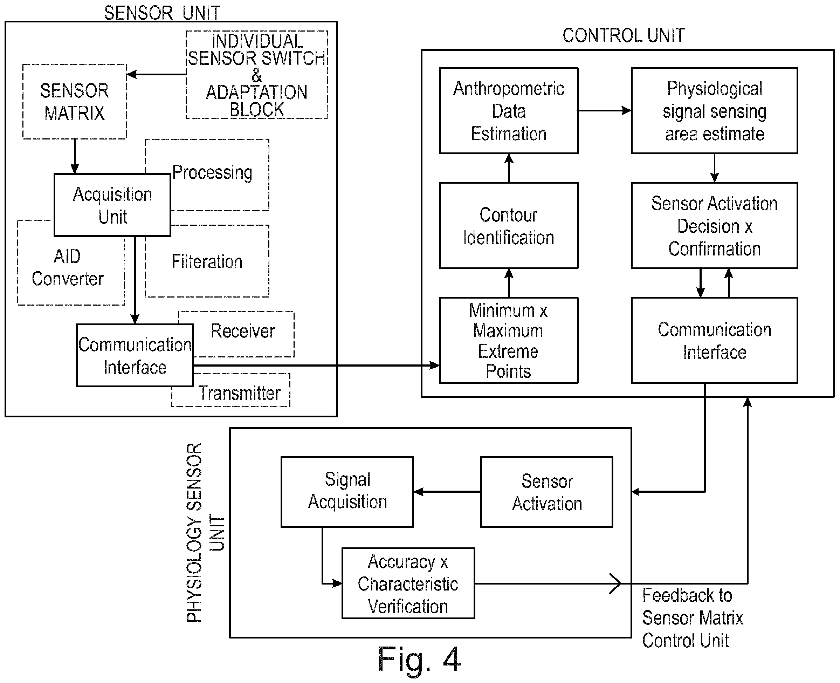

Figure 4 shows how an exemplary control unit processes signals/data and how it may cooperate with an exemplary sensor matrix and an exemplary physiology sensor unit, as a block diagram.

In the exemplary sensor matrix, individual sensors can be switched and may be adapted to their respective purpose (S11). Signals from the sensor matrix can be received by an acquisition unit (S12) which passes on acquired signals to a communication interface. The acquired signals may be converted into digital signals and/or can be filtered into processed signals (S12). The sensor unit's communication interface serves to transfer the signals/data to the control unit (S13). The sensor unit can have a receiver and a transmitter for communicating with the control unit.

The exemplary control unit is arranged to determine minimum and maximum extreme points (S 14). These extreme points can be used for contour identification (S 15), anthropometric data estimation (S 16) and physiological signal sensing area estimation (S17). Based on the physiological signal sensing area estimation, sensor activation can be decided and sensor configuration can be instructed by the control unit (S 18). The control unit can communicate the instructions to the exemplary physiology sensor unit (S 19).

Based on these instructions, sensors are activated by the exemplary physiology sensor unit (S20). Afterwards, signal acquisition occurs by the activated sensors (S21). The accuracy and characteristics of the acquired data can be verified by the exemplary physiology sensor unit (evaluation) (S22). The evaluation can be fed to a sensor matrix control unit and/or to the exemplary control unit (S23). The feedback can be used in the control unit for decisions on sensor activation and instructions for sensor configuration.

### Reference signs

- 1: smart cover
- 2: sheet of fabric or leather
- 3: sensor matrix
- 4: sensor element
- 11: independent car seat
- 21: anthropometric data of occupant
- 22: physiologic parameter of occupant
- 23: occupant presence information
- 24: evaluated physiologic parameter, such as ECG, GSR, EEG

## Claims

1. Smart cover (1) for sensing a signal, the smart cover comprising
a preferably conducting sheet (2) of fabric or leather arranged to cover a seat, a handle and/or a steering device particularly of a vehicle, and
a sensor matrix (3) printed on the sheet and comprising several sensor elements (4), wherein each of the sensor elements is arranged for sensing or detecting electrical signal sent out by a body of a human occupant, an outline of the body, and a body dimension, whereby the sensor matrix (3) is arranged to detect the occupant's physique, and is arranged to provide anthropometric data (21), based on the signals received by the sensor elements (4) [original Claim 3], whereby an electronic control unit is arranged to process signals from the sensor elements (4) into anthropometric data (21), a physiologic parameter (22) and an ECG, GSR or EEG (24) (evaluated physiologic parameter) of the occupant [original Claim 7].

2. Smart cover (1) according to claim 1, **characterised in that** the sensor matrix is made with capacitive sensor elements (4).

3. Smart cover (1) according to one of the preceding claims, **characterised in that** a first group of sensor elements is positioned and can be activated to detect electric heart signals of the occupant.

4. Smart cover (1) according to one of the preceding claims, **characterised in that** the sheet (2) is arranged to cover the handle or the steering device, and **in that** some of the sensor elements (4) are positioned and arranged to detect or provide a dual electrode ECG from the occupant's fingers placed on the handle or steering device, particularly from the fingers of the right & left hand placed on the handle or steering device.

5. Smart cover (1) according to one of claims 2 to 4, **characterised in that** the sheet is arranged to cover the seat, and **in that** some of the sensor elements (4) are positioned and arranged to detect or provide a 12 electrode ECG by estimating the location of exact electrode positions from the electrically generated profile or anthropometric data.

6. Vehicle having a smart cover (1) according to one of the preceding claims and a safety system, **characterised in that** the smart cover (1) is connected with the safety system to provide anthropometric data (21) and a physiologic parameter (22) of the occupant to the safety system, preferably **characterised in that** the safety system is arranged to operate based on the anthropometric data and the physiologic parameter.

7. System **characterised by** a smart cover according to one of claims 1 to 5 and a piece of gym equipment having a seat and/or a handle, the smart cover (1) being arranged to cover the seat or handle.

8. Method for operating a smart cover (1), particularly according to one of the preceding claims, the method comprising the steps:
S2 sensing or detecting by sensor elements (4) an electrical signal sent out by a body of a human occupant, an outline of the body, and a body dimension, wherein the sensor elements (4) are arranged in a sensor matrix (3) of the smart cover (1), which sensor matrix is printed on a preferably conductive sheet (2) of fabric or leather of the smart cover, wherein the sheet is arranged to cover a seat, handle or a steering device particularly of a vehicle, particularly to detect the presence of the occupant and/or to provide occupant presence information (21),
further comprising
S3 estimating the physique of the occupant based on the signals received by the sensor elements (4), and providing respective anthropometric data (21), and
S5 increasing the sensitivity of at least one of the sensor elements according to data of step S3, [original Clam 13] and
S6' obtaining a physiologic parameter of the occupant, namely ECG, GSR or EEG, based on the signals received by the sensor elements, using the at least one sensor element of step S5 [original Claim 13].

9. Method according to claim 8, **characterised by** the following step
S1 activating the smart cover (1), particularly when switching on an ignition device of a vehicle.

10. Method according to one of claims 8 9, **characterised by** step
S4 activating or adjusting an adaptive passive safety system of a vehicle based on the anthropometric data of step S3.

11. Method according to one of claims 8 to 10, **characterised by** step
S7 activating a predictive safety system and/or a comfort system based on the physiologic parameter of step S6 or step S6'.

## Patentansprüche

1. Intelligente Abdeckung (1) zum Erfassen eines Signals, wobei die intelligente Abdeckung Folgendes aufweist:
eine vorzugsweise leitende Lage (2) aus Stoff oder Leder, die ausgelegt ist, einen Sitz, einen Griff und/oder eine Lenkvorrichtung insbesondere eines Fahrzeugs abzudecken, und
eine Sensormatrix (3), die auf die Lage gedruckt ist und mehrere Sensorelemente (4) aufweist, wobei jedes der Sensorelemente zum Erfassen oder Detektieren eines elektrischen Signals, das von einem Körper eines menschlichen Insassen gesendet wurde, eines Umrisses des Körpers und einer Körperabmessung ausgelegt ist, wodurch die Sensormatrix (3) ausgelegt ist, den Körperbau eines Insassen zu detektieren, und ausgelegt ist, anthropometrische Daten (21) auf der Grundlage der Signale bereitzustellen, die durch die Sensorelemente (4) empfangen wurden [ursprünglich Anspruch 3], wodurch eine elektronische Steuereinheit ausgelegt ist, Signale von den Sensorelementen (4) in anthropometrische Daten (21), einen physiologischen Parameter (22) und ein ECG, GSR oder EEG (24) (einen bewerteten physiologischen Parameter) des Insassen zu verarbeiten [ursprünglich Anspruch 7].

2. Intelligente Abdeckung (1) nach Anspruch 1, **dadurch gekennzeichnet, dass** die Sensormatrix mit kapazitiven Sensorelementen (4) gebildet ist.

3. Intelligente Abdeckung (1) nach einem der vorhergehenden Ansprüche, **dadurch gekennzeichnet, dass** eine erste Gruppe von Sensorelementen positioniert ist und aktiviert werden kann, um elektrische Herzsignale des Insassen zu detektieren.

4. Intelligente Abdeckung (1) nach einem der vorhergehenden Ansprüche, **dadurch gekennzeichnet, dass** die Lage (2) ausgelegt ist, den Griff oder die Lenkvorrichtung abzudecken, und dass ein Teil der Sensorelemente (4) positioniert und ausgelegt ist, ein Doppelelektroden-ECG von den Fingern eines Insassen, die an dem Griff oder der Lenkvorrichtung angeordnet sind, insbesondere von den Fingern der rechten & der linken Hand, die an dem Griff oder der Lenkvorrichtung angeordnet sind, zu detektieren oder bereitzustellen.

5. Intelligente Abdeckung (1) nach einem der Ansprüche 2 bis 4, **dadurch gekennzeichnet, dass** die Lage ausgelegt ist, den Sitz abzudecken, und dass ein Teil der Sensorelemente (4) positioniert und ausgelegt ist, ein 12-Elektroden-ECG durch Schätzen des Orts genauer Elektrodenpositionen aus dem elektrisch erzeugten Profil oder anthropometrischen Daten zu detektieren oder bereitzustellen.

6. Fahrzeug, das eine intelligente Abdeckung (1) nach einem der vorhergehenden Ansprüche und ein Sicherheitssystem besitzt, **dadurch gekennzeichnet, dass** die intelligente Abdeckung (1) mit dem Sicherheitssystem verbunden ist, um anthropometrische Daten (21) und einen physiologischen Parameter (22) des Insassen zum Sicherheitssystem zu liefern, und vorzugsweise **dadurch gekennzeichnet, dass** das Sicherheitssystem ausgelegt ist, auf der Grundlage der anthropometrischen Daten und des physiologischen Parameters zu arbeiten.

7. System, **gekennzeichnet durch** eine intelligente Abdeckung nach einem der Ansprüche 1 bis 5 und einen Teil eines Fitnessstudiogeräts, das einen Sitz und/oder einen Griff besitzt, wobei die intelligente Abdeckung (1) ausgelegt ist, den Sitz oder den Griff abzudecken.

8. Verfahren zum Betreiben einer intelligenten Abdeckung (1), insbesondere nach einem der vorhergehenden Ansprüche, wobei das Verfahren die folgenden Schritte umfasst:
S2 Erfassen oder Detektieren durch Sensorelemente (4) eines elektrischen Signals, das von einem Körper eines menschlichen Insassen ausgesendet wird, eines Umrisses des Körpers und einer Körperabmessung, wobei die Sensorelemente (4) in einer Sensormatrix (3) der intelligenten Abdeckung (1) angeordnet sind, die Sensormatrix auf eine vorzugsweise leitende Lage (2) eines Stoffs oder von Leder der intelligenten Abdeckung gedruckt ist und die Lage ausgelegt ist, einen Sitz, einen Griff oder eine Lenkvorrichtung insbesondere eines Fahrzeugs abzudecken, um insbesondere das Vorliegen des Insassen zu detektieren und/oder Insassenanwesenheitsinformationen (21) bereitzustellen, und ferner Folgendes aufweist:
S3 Schätzen des Körperbaus des Insassen auf der Grundlage der Signale, die durch die Sensorelemente (4) empfangen werden, und Bereitstellen jeweiliger anthropometrischer Daten (21), und
S5 Erhöhen der Empfindlichkeit mindestens eines der Sensorelemente gemäß Daten von Schritt S3, [ursprünglich Anspruch 13] und
S6' Erhalten eines physiologischen Parameters des Insassen, und zwar eines ECG, GSR oder EEG, auf der Grundlage der Signale, die durch die Sensorelemente unter Verwendung des mindestens einen Sensorelements von Schritt S5 empfangen wurden [ursprünglich Anspruch 13].

9. Verfahren nach Anspruch 8, **gekennzeichnet durch** den folgenden Schritt:
S1 Aktivieren der intelligenten Abdeckung (1), insbesondere wenn an einer Zündvorrichtung eines Fahrzeugs geschaltet wird.

10. Verfahren nach einem der Ansprüche 8 und 9, **gekennzeichnet durch** den folgenden Schritt:
S4 Aktivieren oder Einstellen eines adaptiven passiven Sicherheitssystems eines Fahrzeugs auf der Grundlage der anthropometrischen Daten von Schritt S3.

11. Verfahren nach einem der Ansprüche 8 bis 10, **gekennzeichnet durch** den folgenden Schritt:
S7 Aktivieren eines vorausschauenden Sicherheitssystems und/oder eines Komfortsystems auf der Grundlage des physiologischen Parameters von Schritt S6 oder Schritt S6'.

## Revendications

1. Housse intelligente (1) pour détecter un signal, la housse intelligente comprenant
une feuille de préférence conductrice (2) de tissu ou de cuir conçue pour recouvrir un siège, un guidon et/ou un dispositif de direction en particulier d'un véhicule, et une matrice (3) de capteurs imprimée sur la feuille et comprenant plusieurs éléments capteurs (4), chacun des éléments capteurs étant conçu pour capter ou détecter un signal électrique émis par un corps d'un occupant humain, un contour du corps, et une dimension corporelle, la matrice (3) de capteurs étant conçue pour détecter le physique de l'occupant et étant conçue pour fournir des données anthropomorphiques (21) sur la base de signaux reçus par les éléments capteurs (4) [Revendication 3 à l'origine], moyennant quoi une unité de commande électronique est conçue pour traiter des signaux provenant des éléments capteurs (4) pour produire des données anthropométriques (21), un paramètre physiologique (22) et une ECG, une GSR ou une EEG (24) (paramètre physiologique évalué) de l'occupant [Revendication 7 à l'origine].

2. Housse intelligente (1) selon la revendication 1, **caractérisée en ce que** la matrice de capteurs est constituée d'éléments capteurs capacitifs (4).

3. Housse intelligente (1) selon l'une des revendications précédentes, **caractérisée en ce qu'**un premier groupe d'éléments capteurs est positionné et est activable pour détecter des signaux électriques cardiaques de l'occupant.

4. Housse intelligente (1) selon l'une des revendications précédentes, **caractérisée en ce que** la feuille (2) est conçue pour recouvrir le guidon ou le dispositif de direction, et **en ce que** certains des éléments capteurs (4) sont positionnés et conçus pour détecter ou fournir une ECG à deux électrodes à partir des doigts de l'occupant placés sur le guidon ou le dispositif de direction, en particulier à partir des doigts de la main droite et de la main gauche placées sur le guidon ou le dispositif de direction.

5. Housse intelligente (1) selon l'une des revendications 2 à 4, **caractérisée en ce que** la feuille est conçue pour recouvrir le siège, et **en ce que** certains des éléments capteurs (4) sont positionnés et conçus pour détecter ou fournir une ECG à 12 électrodes en estimant l'emplacement de positions exactes des électrodes à partir du profil généré électriquement ou des données anthropométriques.

6. Véhicule équipé d'une housse intelligente (1) selon l'une des revendications précédentes et d'un système de sécurité, **caractérisé en ce que** la housse intelligente (1) est connectée au système de sécurité pour fournir des données anthropométriques (21) et un paramètre physiologique (22) de l'occupant au système de sécurité, de préférence **caractérisé en ce que** le système de sécurité est conçu pour fonctionner sur la base des données anthropométriques et du paramètre physiologique.

7. Système **caractérisé par** une housse intelligente selon l'une des revendications 1 à 5 et un appareil de salle de sport équipé d'un siège et/ou d'un guidon, la housse intelligente (1) étant conçue pour recouvrir le siège ou le guidon.

8. Procédé pour faire fonctionner une housse intelligente (1), en particulier selon l'une des revendications précédentes, le procédé comprenant les étapes suivantes :
S2 captage ou détection par des éléments capteurs (4) d'un signal électrique émis par un corps d'un occupant humain, d'un contour du corps, et d'une dimension corporelle, les éléments capteurs (4) étant agencés dans une matrice (3) de capteurs de la housse intelligente (1), laquelle matrice de capteurs est imprimée sur une feuille de préférence conductrice (2) de tissu ou de cuir, la feuille étant conçue pour recouvrir un siège, un guidon ou un dispositif de direction en particulier d'un véhicule, en particulier pour détecter la présence de l'occupant et/ou pour fournir des informations (21) sur la présence de l'occupant, comprenant en outre
S3 estimation du physique de l'occupant sur la base des signaux reçus par les éléments capteurs (4), et fourniture de données anthropométriques (21) respectives, et
S5 accroissement de la sensibilité d'au moins un des éléments capteurs selon les données de l'étape S3, [Revendication 13 à l'origine] et
S6' obtention d'un paramètre physiologique de l'occupant, à savoir d'une ECG, d'une GSR ou d'une EEG, sur la base des signaux reçus par les éléments capteurs, à l'aide de l'au moins un élément capteur de l'étape S5 [Revendication 13 à l'origine].

9. Procédé selon la revendication 8, **caractérisé par** l'étape suivante
S1 activation de la housse intelligente (1), en particulier lors de la mise sous tension d'un dispositif d'allumage d'un véhicule.

10. Procédé selon l'une des revendications 8 et 9, **caractérisé par** l'étape suivante
S4 activation ou réglage d'un système de sécurité passif adaptatif d'un véhicule sur la base des données anthropomorphiques de l'étape S3.

11. Procédé selon l'une des revendications 8 à 10, **caractérisé par** l'étape suivante
S7 activation d'un système de sécurité prédictif et/ou d'un système de confort sur la base du paramètre physiologique de l'étape S6 ou de l'étape S6'.
